# EUROPÄISCHE PATENTSCHRIFT

(11) **EP 0 717 029 B1**
(45) Veröffentlichungstag und Bekanntmachung des Hinweises auf die Patenterteilung: **13.10.1999**
(21) Anmeldenummer: 95118901.8
(22) Anmeldetag: 01.12.1995
(51) Int. Cl.: C07C 57/04, C07C 51/44

(54) **Verfahren der rektifikativen Abtrennung von (Meth)acrylsäure aus einem hydrophobe Roh(meth)acrylsäure enthaltenden Gemisch**
Process for a rectificative separation of (meth)acrylic acid from a hydrophobic mixture containing crude (meth)acrylic acid
Procédé de séparation rectificative d'acide (méth)acrylique à partir d'un mélange hydrophobe contenant de l'acide (méth)acrylique brut

(30) Priorität: 14.12.1994 US 355892
(43) Veröffentlichungstag der Anmeldung: 19.06.1996
(73) Patentinhaber: BASF Aktiengesellschaft, 67063 Ludwigshafen (DE)
(72) Erfinder: Herbst, Holger, Dr., D-67227 Frankenthal (DE); Nestler, Gerhard, Dr., D-67061 Ludwigshafen (DE); Hammon, Ulrich, Dr., D-68165 Mannheim (DE); Darlington, Jerry, Lake Jackson, Texas 77566 (US)

(56) Entgegenhaltungen:
- EP-A- 0 312 191
- EP-A- 0 685 448
- DE-A- 2 136 396
- DE-A- 2 207 184
- DE-A- 2 235 326
- US-A- 3 725 208

## Beschreibung

Die vorliegende Erfindung betrifft ein neues Verfahren der rektifikativen Abtrennung von (Meth)acrylsäure aus einem (Meth)acrylsäure und eine hoher als (Meth)acrylsäure siedende inerte hydrophobe organische Flüssigkeit als Hauptbestandteile sowie niedere Aldehyde als Nebenbestandteile enthaltenden Gemisch.

(Meth)acrylsäure wird als verkürzte Schreibweise verwendet und steht für Acrylsäure oder Methacrylsäure.

(Meth)acrylsäure, entweder für sich oder in Form ihrer Ester, ist insbesondere zur Herstellung von Polymerisaten für die verschiedensten Anwendungsgebiete, z.B. Verwendung als Klebstoffe, von Bedeutung.

Unter anderem ist (Meth)acrylsäure durch katalytische Gasphasenoxidation von Alkanen, Alkanolen, Alkenen oder Alkenalen erhältlich, die 3 bzw. 4 C-Atome enthalten. Besonders vorteilhaft ist (Meth)acrylsäure z.B. durch katalytische Gasphasenoxidation von Propen, Acrolein, tert.-Butanol, iso-Buten, iso-Butan, iso-Butyraldehyd oder Methacrolein erhältlich. Als Ausgangsverbindungen sind aber auch solche denkbar, aus welchen sich die eigentliche C₃-/C₄-Ausgangsverbindung während der Gasphasenoxidation erst intermediär bildet. Beispielhaft genannt sei der Methylether des tert.-Butanols.

Dabei werden diese Ausgangsgase, in der Regel mit Inertgasen wie Stickstoff, CO, CO₂, gesättigten Kohlenwasserstoffen und/oder Wasserdampf verdünnt, im Gemisch mit Sauerstoff bei erhöhten Temperaturen (üblicherweise 200 bis 400°C) sowie gegebenenfalls erhöhtem Druck über übergangsmetallische (z.B. Mo, V, W und/oder Fe enthaltende) Mischoxidkatalysatoren geleitet und oxidativ in die (Meth)acrylsäure umgewandelt (vgl. z.B. DE-A 4 405 059, EP-A 253 409, EP-A 92 097, DE-A 44 31 957 und DE-A 44 31 949).

Aufgrund zahlreicher im Verlauf der katalytischen Gasphasenoxidation erfolgender Parallel- und Folgereaktionen sowie aufgrund der mitzuverwendenden inerten Verdünnungsgase wird bei der katalytischen Gasphasenoxidation jedoch keine reine (Meth)acrylsäure sondern ein Reaktionsgasgemisch erhalten, das im wesentlichen (Meth)acrylsäure, die inerten Verdünnungsgase und Nebenprodukte enthält, aus welchem die (Meth)acrylsäure abgetrennt werden muß. Neben von (Meth)acrylsäure vergleichsweise einfach zu entfernenden und bei Folgeverwendungen der (Meth)acrylsäure weniger störenden Nebenprodukten wie z.B. Essigsäure, enthält das Reaktionsgasgemisch insbesondere auch mit (Meth)acrylsäure eng verwandte und daher von (Meth)acrylsäure schwer abtrennbare niedere Aldehyde wie Formaldehyd, Acetaldehyd, Acrolein, Methacrolein, Propionaldehyd, n-Butyraldehyd, Benzaldehyd, Furfural und Crotonaldehyd sowie zusätzich gegebenenfalls Maleinsäureanhydrid (bezogen auf die im Reaktionsgasgemisch enthaltene Menge an (Meth)acrylsäure beträgt die Gesamtmenge dieser, bei Folgeverwendungen häufig erheblich störenden, Nebenkomponenten in der Regel < 2 Gew.-%).

Die DE-A 44 36 243 betrifft ein Verfahren zur Abtrennung von (Meth)acrylsäure aus dem Reaktionsgasgemisch der katalytischen Gasphasenoxidation durch Gegenstromabsorption mit einer hochsiedenden inerten hydrophoben organischen Flüssigkeit, bei dem man das Reaktionsgasgemisch in einer Absorptionskolonne zu der absteigenden hochsiedenden inerten hydrophoben organischen Flüssigkeit im Gegenstrom führt, dem in der Absorptionskolonne in natürlicher Weise erfolgenden Absorptionsprozeß einen Rektifikationsprozeß überlagert, indem man der Absorptionskolonne eine über ihre, aufgrund ihres Kontaktes mit der Umgebungstemperatur erfolgende, natürliche Energieabgabe hinausgehende Energiemenge entzieht, und aus dem (Meth)acrylsäure und das Absorptionsmittel (Absorbens) als Hauptbestandteile sowie niedere Aldehyde und gegebenenfalls Maleinsäureanhydrid als Nebenbestandteile enthaltenden Flüssigkeitsablauf der Absorptionskolonne (Absorbat) die (Meth)acrylsäure rektifikativ abtrennt. Die dabei erhältiche (Meth)acrylsäure wird als Roh-(Meth)acrylsäure bezeichnet. Sie weist in der Regel eine Reinheit > 98 Gew.-% auf, wobei sich die Verunreinigungen insbesondere aus den benannten niederen Aldehyden und gegebenenfalls Maleinsäureanhydrid rekrutieren, wohingegen die Trennung der (Meth)acrylsäure von der hochsiedenden inerten organischen Absorptionsflüssigkeit im wesentlichen quantitativ erfolgt ist.

Als hochsiedende inerte hydrophobe organische Flüssigkeit (Absorbens) faßt die DE-A 44 36 243 dabei alle diejenigen Flüssigkeiten zusammen, deren Siedetemperatur bei Normaldruck (1 atm) oberhalb der Siedetemperatur der (Meth)acrylsäure liegt und die zu wenigstens 70 Gew.-% aus solchen Molekülen bestehen, die keine nach außen wirkende polare Gruppe enthalten und somit beispielsweise nicht in der Lage sind, Wasserstoffbrücken zu bilden. Dieser Begriffsinhalt gilt auch hier.

Aus der DE-PS 2 136 396 und der DE-A 43 08 087 ist ebenfalls bekannt, Acrylsäure aus dem Reaktionsgasgemisch der katalytischen Gasphasenoxidation von Propylen und/oder Acrolein durch Gegenstromabsorption mit einer hochsiedenden inerten hydrophoben organischen Flüssigkeit abzutrennen. Das Verfahren wird im wesentlichen so durchgeführt, daß man das Reaktionsgasgemisch in einer konventionellen Absorptionskolonne zu der absteigenden Absorptionsflüssigkeit im Gegenstrom führt, danach in einer Desorptionskolonne aus dem im wesentlichen aus Acrylsäure, dem Absorptionsmittel und Nebenkomponenten zusammengesetzten Flüssigkeitsablauf der Absorptionskolonne durch Strippen (Abstreifen) mit Inertgas die einfach abtrennbaren leichtflüchtigen Nebenkomponenten weitgehend entfernt und anschließend den Acrylsäure und das Absorbens als Hauptbestandteile sowie niedere Aldehyde und gegebenenfalls Maleinsäureanhydrid als Nebenbestandteile enthaltenden Flüssigkeitsablauf der Desorptionskolonne zur Abtrennung von Roh-Acrylsäure rektifikativ behandelt.

Nachteilig an der rektifikativen Abtrennung von Roh-(Meth)acrylsäure aus (Meth)acrylsäure und eine hochsiedende inerte hydrophobe organische Flüssigkeit als Hauptbestandteile sowie niedere Aldehyde und gegebenenfalls Maleinsäureanhydrid als Nebenbestandteile enthaltenden Gemischen ist jedoch, daß sich im Verlauf der Rektifikation trotz Mitverwendung an sich üblicher Mengen üblicher Polymerisationsinhibitoren wie Phenothiazin, Paramethoxyphenol, Paranitrosophenol, Hydrochinon, Hydrochinonmonomethylether oder Luft die Rektifikationsvorrichtungen (insbesondere die Verdampferoberfläche und die Kolonneneinbauten) mit Belag bedecken. Wird die rektifikative Abtrennung kontinuierlich betrieben, weist die unterschiedliche Färbung der Belagsbildung in der Abtriebsäule (schwarz) und der Verstärkersäule (weiß) aus, daß an der Belagsbildung wenigstens zwei Prozesse beteiligt sind. Die Belagsbildung ist insofern von Nachteil, als sie von Zeit zu Zeit entfernt werden muß, was ein Abschalten des Rektifikationsbetriebes bedingt.

Aufgabe der vorliegenden Erfindung war daher ein Verfahren der rektifikativen Abtrennung von (Meth)acrylsäure aus einem (Meth)acrylsäure und eine höher als (Meth)acrylsäure siedende inerte hydrophobe organische Flüssigkeit als Hauptbestandteile sowie niedere Aldehyde als Nebenbestandteile enthaltenden Gemisch, das eine verminderte Belagsbildung und dadurch einen verlängerten Rektifikationsbetrieb ermöglicht.

Demgemäß wurde ein verfahren der rektifikativen Abtrennung von (Meth)acrylsäure aus einem (Meth)acrylsäure und eine höher als (Meth)acrylsäure siedende inerte hydrophobe organische Flüssigkeit als Hauptbestandteile sowie niedere Aldehyde als Nebenbestandteile enthaltenden Gemisch gefunden, das dadurch gekennzeichnet ist, daß die Rektifikation unter Zusatz eines primären Amins und/oder dessen Salzen erfolgt und es sich bei der hydrophoben organischen Flüssigkeit um ein Gemisch bestehend aus einer Mischung aus 70 bis 75 Gew.-% Diphenylether und 25 bis 30 Gew.-% Diphenyl sowie, bezogen auf diese Mischung, 0,1 bis 25 Gew.-% o-Dimethylphthalat handelt. Unter einem primären Amin werden solche Verbindungen verstanden, die wenigstens eine -NH₂-Gruppe aufweisen.

Dabei konnte von nachfolgendem Stand der Technik ausgegangen werden.

Die DE-A 2 235 326 betrifft ein verfahren zur Reinigung von 1,2 ungesättigten Carbonsäuren bei dem 2-Ethylhexanol als bevorzugtes Lösungsmittel mitverwendet wird. Selbiges ist gegenüber Acrylsäure jedoch nicht inert sondern verestert mit Acrylsäure.

Aus der DE-B 22 07 184 und der GB-PS 1 346 737 ist ein Verfahren zur Reinigung von Roh-Acrylsäure bekannt, das dadurch gekennzeichnet ist, daß man der Roh-Acrylsäure wenigstens ein primäres Amin wie z.B. Hydrazin, Phenylhydrazin, Anilin, Monoethanolamin, Ethylendiamin und/oder Glycin zugibt und aus dem Gemisch die Acrylsäure destillativ abtrennt.

Die zugesetzten primären Amine binden dabei offensichtlich die als Verunreinigungen enthaltenen Aldehyde in hohem Ausmaß, so daß bereits eine sich anschließende einfache destillative Trennstufe bezüglich der aldehydischen Verunreinigungen eine hohe Trennwirkung erzielt. Die EP-A 270 999 empfiehlt in entsprechender Weise Roh-(Meth)acrylsäure vor der destillativen Aufarbeitung Guanylhydrazin (Aminoguanidin) und/oder dessen Salze (vorzugsweise Aminoguanidinhydrogencarbonat) zuzusetzen und die U.S. Appl. Ser. No. 08/347,131 (O.Z. 0050/45375, NAE 642/94), lehrt ein Verfahren zur Reinigung einer mit niederen Aldehyden verunreinigten Roh-(Meth)acrylsäure, bei dem man die (Meth)acrylsäure mit einem Carbonsäurehydrazid und/oder dessen Salze versetzt und aus dem Gemisch die (Meth)acrylsäure destillativ abtrennt.

Nachteiligt an diesen Empfehlungen des Standes der Technik zur Abtrennung der niederen Aldehyde aus Roh-(Meth)acrylsäure ist jedoch, daß durch das Beisein der primären Amine bei der destillativen Aufarbeitung der Roh-(Meth)acrylsäure eine verstärkte Belagsbildung auf den Destillationsoberflächen bedingt wird (vgl. z.B. DE-A 43 35 172 und U.S. Appl. Ser. No. 08/347,131 (O.Z. 0050/45375, NAE 642/94), dem man durch zusätzlichen Zusatz einer organischen Sulfonsäure zu begegnen sucht.

Offensichtlich sind an der verstärkten Belagsbildung unmittelbare Umsetzungsprodukte des zugesetzten primären Amins mit den aldehydischen Verunreinigungen und/oder im Rahmen der destillativen Aufarbeitung aus diesen entstehende Folgeprodukte beteiligt.

Überraschend wurde nun gefunden, daß bei dem erfindungsgemäßen Verfahren, das sich von der destillativen (rektifikativen) Behandlung einer Roh-(Meth)acrylsäure insbesondere durch das Beisein der speziellen hochsiedenden inerten hydrophoben organischen Flüssigkeit unterscheidet, die durch das Beisein des primären Amins zu erwartende verstärkte Belagsbildung nicht nur unterbleibt, sondern im Vergleich zu einer Abwesenheit des primären Amins sogar eine verringerte Belagsbildung beobachtet wird. Gleichzeitig wird im Rahmen des erfindungsgemäßen Verfahrens eine Roh-(Meth)acrylsäure erhalten, deren Gehalt an niederen Aldehyden und Maleinsäureanhydrid wesentlich vermindert ist.

Als erfindungsgemäß geeignete zuzusetzende primäre Amine und/oder deren Salze seien beispielhaft genannt (aufgelistet wird der Einfachheit halber lediglich die Aminform; die Salze ergeben sich dazu entsprechend) Hydrazin und dessen Derivate wie z.B. Guanylhydrazin (Aminoguanidin) und Phenylhydrazin, aromatische Amine, die vorzugsweise bis zu 12 C-Atome aufweisen, wie z.B. Anilin, o-, m-, p-Toluidin und o-, m-, p-Nitroanilin, Aminocarbonsäuren wie z.B. Glycin, Aminoalkohole wie z.B. Ethanolamin (2-Aminoethanol), aber auch kettenförmige, verzweigte oder cyclische aliphatische Amine, die 1 bis 12 C-Atome aufweisen wie z.B. Methylamin. Selbstverständlich kommen auch mehrwertige primäre Amine in Betracht. D.h. es eignen sich auch Verbindungen, die mehr als eine, beispielsweise 2, 3 oder 4 -NH₂-Gruppen aufweisen. Beispielhaft genannt seien 1,2-Diaminoethan, Putrescin (Tetramethylendiamin) und Cadaverin (Pentamethylendiamin).

Geeignete Salze der zuzusetzenden primären Amine sind insbesondere deren Hydrogencarbonat, Nitrat, Sulfat oder Chlorid. Beispielhaft genannt sei das Aminoguanidin-Hydrogencarbonat, wobei das Aminoguanidin-Hydrogencarbonat die vorzugsweise zugesetzte Aminoguanidinverbindung ist.

Eine erfindungsgemäß besonders vorteilhafte Gruppe primärer Amine sind die Hydrazide organischer Carbonsäuren. Als solche Hydrazide organischer Carbonsäuren kommen insbesondere in Betracht: Semicarbazid (Carbamidsäurehydrazid) sowie die Mono- und Dihydrazide von 1 bis 10 C-Atome aufweisenden gesättigten aliphatischen Mono- und/oder Dicarbonsäuren. Das sind insbesondere die Hydrazide von Ameisensäure, Essigsäure, Propionsäure, Butansäure und Pentansäure. Als gesättigte aliphatische Dicarbonsäuren kommen für die entsprechenden Hydrazide insbesondere diejenigen in Betracht, die 4 bis 8 C-Atome aufweisen. Ganz besonders geeignet sind das Dihydrazid der Adipinsäure und der Bernsteinsäure. Selbstverständlich können anstelle der Carbonsäurehydrazide auch deren Salze eingesetzt werden. Als solche kommen beispielsweise deren Hydrogencarbonat, Nitrat, Sulfat oder Chlorid in Betracht, wie z.B. Semicarbazidhydrochlorid.

Die Menge des erfindungsgemäß zuzusetzenden primären Amins orientiert sich insbesondere am Aldehydgehalt des Flüssigkeitsgemisches, aus dem die (Meth)acrylsäure rektifikativ abzutrennen ist. Dieser läßt sich in dem Fachmann bekannter Weise, nach geeigneter Derivatisierung der Aldehyde, durch die Methode der Hochdruckflüssigchromatographie (HPLC) ermitteln. Pro Mol aldehydischer Verunreinigungen wird man in der Regel wenigstens 0,5 Mol, normalerweise aber nicht mehr als 5 Mol, an primärem Amin zusetzen. Vorzugsweise beträgt die zuzusetzende Menge an primärem Amin, in gleicher Weise bezogen, 1 bis 3 Mol und ganz besonders bevorzugt 1 bis 2 Mol.

Von besonderer Bedeutung ist das erfindungsgemäße Verfahren im Fall von Methacrylsäure, deren gasphasenkatalytisch oxidative Herstellung ausgehend von Methacrolein erfolgt, insbesondere dann, wenn das Methacrolein durch gasphasenkatalytische Oxidation von tert.-Butanol, iso-Butan oder iso-Buten oder durch Umsetzung von Formaldehyd mit Pripionaldehyd gemäß der EP-B 92 097 oder der EP-B 58 927 erzeugt wird, und dieses insbesondere dann, wenn die gasphasenkatalytische Oxidation des tert.-Butanols, iso-Butans oder iso-Butens unter Verwendung einer katalytisch aktiven Masse der allgemeinen Formel I

Mo₁₂BiₐFe_{b}X¹_{c}X²_{d}X³ₑX⁴_{f}Oₙ (I),

in der die Variablen folgende Bedeutung haben:
- X¹: Nickel und/oder Kobalt,
- X²: Thallium ein Alkalimetall und/oder ein Erdalkalimetall,
- X³: Phosphor, Arsen, Bor, Antimon, Zinn, Cer, Blei, Niob und/oder Wolfram,
- X⁴: Silicium, Aluminium, Titan und/oder Zirkonium,
- a: 0,5 bis 5,
- b: 0,01 bis 3,
- c: 3 bis 10,
- d: 0,02 bis 2,
- e: 0 bis 5,
- f: 0 bis 10 und
- n: eine ganze Zahl, die durch die Wertigkeit und Häufigkeit der von Sauerstoff verschiedenen Elemente in I bestimmt wird,
bei Temperaturen von 300 bis 400°C und, vom speziellen Temperaturverlauf abgesehen, ansonsten gemäß den Bedingungen der DE-A 40 23 239 erfolgt und das anfallende Methacrolein ohne Zwischenreinigung zur Weiteroxidation verwendet wird. Ferner bewährt sich das erfindungsgemäße Verfahren besonders dann, wenn die gasphasenkatalytische Oxidation des Methacroleins, vom speziellen Temperaturverlauf abgesehen, gemäß den Ausführungen der DE-A 41 32 263 bei Temperaturen von 200 bis 350°C bzw. gemäß der DE-A 41 32 684 bei Temperaturen von 250 bis 400°C erfolgt.

Ferner eignet sich das erfindungsgemäße Verfahren insbesondere im Fall von Acrylsäure, deren gasphasenoxidative Herstellung einstufig ausgehend von Acrolein oder zweistufig ausgehend von Propylen über Acrolein erfolgt. Dies gilt insbesondere dann, wenn für die katalytische Gasphasenoxidation des Propylens ein Multimetalloxidkatalysator der allgemeinen Formel II

Mo₁₂BiₐFe_{b}X¹_{c}X²_{d}X³ₑX⁴_{f}Oₙ (II),

in der die Variablen nachfolgende Bedeutung aufweisen:
- X¹: Nickel und/oder Kobalt,
- X²: Thallium, ein Alkalimetall und/oder ein Erdalkalimetall,
- X³: Phosphat, Arsen, Bor, Antimon, Zinn, Cer, Blei und/oder Wolfram,
- X⁴: Silicium, Aluminium, Titan und/oder Zirkonium,
- a: 0,5 bis 5,
- b: 0,01 bis 3,
- c: 3 bis 10,
- d: 0,02 bis 2,
- e: 0 bis 5,
- f: 0 bis 10 und
- n: eine ganze Zahl, die durch die Wertigkeit und Häufigkeit der von Sauerstoff verschiedenen Elemente bestimmt wird,
und für die katalytische Gasphasenoxidation des Acroleins ein Multimetalloxidkatalysator der allgemeinen Formel III

Mo₁₂VₐW_{b}Cu_{c}Ni_{d}X¹ₑX²_{f}X³_{g}X⁴ₕX⁵ᵢOₙ (III),

in der die Variablen nachfolgende Bedeutung aufweisen:
- X¹: ein oder mehrere Alkalimetalle,
- X²: ein oder mehrere Erdalkalimetalle,
- X³: Chrom, Mangan, Cer, und/oder Niob,
- X⁴: Antimon und/oder Wismut,
- X⁵: Silicium, Aluminium, Titan und/oder Zirkonium,
- a: 1 bis 6,
- b: 0,2 bis 4,
- c: 0,5 bis 6,
- d: 0,2 bis 6,
- e: 0 bis 2,
- f: 0 bis 3,
- g: 0 bis 5,
- h: 0 bis 40,
- i: 0 bis 40 und
- n: eine ganze Zahl, die durch die Wertigkeit und Häufigkeit der von Sauerstoff verschiedenen Elemente bestimmt wird,
verwendet wird. Die Reaktionsgase der ersten Oxidationsstufe werden üblicherweise ohne Zwischenreinigung der zweiten Oxidationsstufe zugeführt.

Die üblicherweise angewendeten Reaktionsbedingungen können z.B. der DE-A 44 31 957 sowie der DE-A 44 31 949 entnommen werden.

Das erfindungsgemäße Verfahren ist insbesondere dann günstig, wenn das (Meth)acrylsäure und die höher als (Meth)acrylsäure siedende inerte hydrophobe organische Flüssigkeit als Hauptbestandteile sowie niedere Aldehyde als Nebenbestandteile enthaltende Gemisch aus den Reaktionsgasgemischen der vorgenannten Gasphasenoxidationen als Flüssigkeitsablauf einer Gegenstromabsorption mit anschließender Desorption durch Abstreifen mit einem Inertgas gemäß der DE-PS 21 36 396 oder der DE-A 43 08 087 oder als Flüssigkeitsablauf einer Gegenstromabsorption mit überlagerter Rektifikation gemäß der DE-A 44 36 243 erhalten wurde.

Dabei kommen als erfindungsgemäße hochsiedende inerte hydrophobe organische Absorptionsflüssigkeiten insbesondere alle diejenigen in Betracht, deren Siedepunkt bei Normaldruck oberhalb von 160°C liegt.

Häufig wird die hochsiedende inerte hydrophobe organische Flüssigkeit in der Absorptionskolonne dabei in solchen Mengen eingesetzt, daß der Flüssigkeitsablauf 5 bis 25, meist 5 bis 15 Gew.-% (Meth)acrylsäure enthält.

Vorzugsweise wird die erfindungsgemäße rektifikative Abtrennung von (Meth)acrylsäure bei reduziertem Druck, zweckmäßigerweise einem Kopfdruck ≤ 100 mbar, in der Regel 10 bis 100 mbar, vorgenommen. In entsprechender Weise betragen die Sumpftemperaturen 100 bis 220°C. Sie kann jedoch auch bei Drücken bis zu 1 bar durchgeführt werden.

Mit Vorteil erfolgt die erfindungsgemäße rektifikative Abtrennung von (Meth)acrylsäure kontinuierlich, wobei die (Meth)acrylsäure über Kopf oder Seitenabzug der Rektifikationskolonne bezogen wird. Das erfindungsgemäß zuzusetzende primäre Amin wird dabei mit Vorteil kurz unterhalb der Entnahmestelle der (Meth)acrylsäure der Rektifikationskolonne zugeführt. Bemerkenswerterweise bewirkt die erfindungsgemäße Verfahrensweise sowohl eine Minderung der Belagsbildung im Verstärkungsteil als auch im Abtriebsteil der Rektifikationskolonne.

Als Rektifikationskolonne kommen alle gängigen Typen in Betracht. D.h. die Rektifikationskolonne kann z.B. eine Glockenboden-, Füllkörper- oder Packungskolonne sein. Vorzugsweise wird eine Glockenbodenkolonne angewendet. Mit Vorteil befindet sich die Stelle der kontinuierlichen Zufuhr des Flüssigkeitsgemisches, aus welchem die (Meth)acrylsäure rektifikativ abzutrennen ist, von der untersten theoretischen Trennstufe aus betrachtet etwa am Ende des ersten Drittels der Strecke zwischen unterster und höchstgelegener theoretischer Trennstufe.

Die im Rahmen der erfindungsgemäßen rektifikativen Abtrennung anfallende Sumpfflüssigkeit kann bei einer kontinuierlichen Ausführungsweise kontinuierlich entnommen und z.B. unmittelbar als Absorptionsflüssigkeit in der vorgeschalteten Absorptionsstufe wiederverwendet werden. Um die Betriebszeiten der Anlage zu erhöhen ist es dabei gegebenenfalls empfehlenswert, einen Teilstrom dieser hochsiedenden organischen Flüssigkeit abzuzweigen und erst nach Abtrennung des Absorptionsmittels in einer Aufarbeitungsstufe rückzuführen. Noch besser wird die gesamte Sumpfflüssigkeit vor ihrer Rückführung in die Absorptionskolonne durch eine solche Aufarbeitungsstufe geführt. Selbstverständlich kann das erfindungsgemäß zuzusetzende primäre Amin bereits in der die Abtrennung der (Meth)acrylsäure aus dem Reaktionsgasgemisch der katalytischen Gasphasenoxidation bewirkenden Absorptionskolonne oder in der gegebenenfalls nachgeschalteten Desorptionskolonne zugesetzt werden, so daß eine direkte Zugabe in die Rektifikationskolonne teilweise oder vollständig entfällt. Wie bereits erwähnt, erfolgt das erfindungsgemäße Verfahren im Beisein üblicher Mengen an üblichen Polymerisationsinhibitoren, vorzugsweise Phenothiazin. Normalerweise werden sie auf die (Meth)acrylsäuremenge (Gewicht) bezogen in Mengen von 50 bis 1000 ppm eingesetzt.

Das erfindungsgemäße Verfahren liefert eine Roh-(Meth)acrylsäure, die besonders arm an niederen aldehydischen Verunreinigungen ist. Die mögliche Betriebsdauer bei kontinuierlichem Betrieb ist signifikant erhöht.

### Beispiel

### (Es wurde in Anwesenheit von 200 ppm (bezogen auf das Gewicht der Acrylsäure) Phenothiazin als Polymerisationsinhibitor gearbeitet.)

Durch katalytische Gasphasenoxidation von Acrolein gemäß Beispiel B1 der DE-A 43 02 991 wurde ein Acrylsäure enthaltendes Reaktionsgasgemisch erzeugt. 2,1 Nm³/l dieses Reaktionsgasgemisches wurden in einem Gaskühler durch Einspritzen eines Kühlmittelgemisches aus 57,4 Gew.-% Diphenylether, 20,7 Gew.-% Diphenyl und 20 Gew.-% o-Dimethylphthalat auf 170°C gekühlt. Anschließend wurde in einem Abscheider der flüssig gebliebene Anteil des Kühlmittels von der aus Reaktionsgas und verdampftem Kühlmittel bestehenden Gasphase abgetrennt. Die eine Temperatur von 170°C aufweisende Gasphase wurde unterhalb des ersten Bodens in eine Glockenbodenkolonne mit 27 Böden eines Durchmessers von 80 mm geleitet und dem Gegenstrom von 3 ltr/h des gleichfalls aus 57,4 Gew.-% Diphenylether, 20,7 Gew.-% Diphenyl und 20 Gew.-% o-Dimethylphthalat zusammengesetzten, am Kolonnenkopf mit einer Temperatur von 45°C aufgegebenen, Absorptionsmittels ausgesetzt. Der Ablauf der Absorptionskolonne wurde in einem Wärmetauscher indirekt auf 105°C erwärmt und auf den Kopf einer Desorptionskolonne gegeben, die als Glockenbodenkolonne mit 20 Böden ausgeführt war. In der Desorptionskolonne wurden leicht abtrennbare im Vergleich zu Acrylsäure leichtsiedende Komponenten wie Essigsäure durch Strippen mit Stickstoff (400 l/h, Gegenstrom) weitgehend aus dem ansonsten Acrylsäure/niedere Aldehyde/Absorptionsmittel enthaltenden Gemisch entfernt. Der Ablauf der Desorptionskolonne wurde auf der Höhe des zehnten Bodens in eine 35 Glockehböden umfassende (Durchmesser der Kolonne: 80 mm) Rektifikationskolonne geleitet und die Acrylsäure unter Vakuum auf der Höhe des sechsundzwanzigsten Glockenbodens in einer Reinheit von 98,5 Gew.-% kontinuierlich entnommen. Die Sumpftemperatur der Rektifikationskolonne betrug 160°C. Der Kopfdruck lag bei 80 mbar, der Sumpfdruck lag bei 100 mbar.

Verglichen wurden die möglichen Betriebsdauern der Rektifikationskolonne ohne und im Beisein von Adipinsäuredihydrazid. Bei Mitverwendung von Adipinsäuredihydrazid (ADH) wurde selbiges der Rektifikationskolonne unmittelbar unterhalb der Entnahmestelle der Roh-Acrylsäure in einer solchen Menge zugesetzt, daß im Verstärkungsteil ein ADH-Gehalt von 400 ppm (bezogen auf das Gesamtgewicht) vorlag.

### Ergebnis:

- a) Abtriebsteil:: ohne ADH betrug die maximale Betriebsdauer 100 h; danach mußte zur Belagsentfernung abgeschaltet werden; mit ADH wurde eine Betriebsdauer von 230 h erreicht;
- b) Verstärkungsteil:: ohne ADH betrug die maximale Betriebsdauer 50 h; danach mußte zur Belagsentfernung abgeschaltet werden; mit ADH wurde eine Betriebsdauer > 230 h erreicht.

Der Gehalt der rektifikativ abgetrennten Roh-Acrylsäure an niederen Aldehyden betrug mit 400 ppm (bezogen auf das Gewicht der Roh-Acrylsäure) weniger als ein Drittel im Vergleich zum auf Acrylsäure bezogenen Gehalt an niederen Aldehyden des der Rektifikationskolonne zugeführten Flüssigkeitsgemisches.

Wurde das ADH bereits vollständig in der Desorptionskolonne zugesetzt, war die Betriebsdauer im Abtriebsteil im Vergleich zur unmittelbaren vollständigen ADH-Zugabe in die Rektifikationskolonne um 100 % erhöht.

## Patentansprüche

1. Verfahren der rektifikativen Abtrennung von (Meth)acrylsäure aus einem (Meth)acrylsäure und eine höher als (Meth)acrylsäure siedende inerte hydrophobe organische Flüssigkeit als Hauptbestandteile sowie niedere Aldehyde als Nebenbestandteile enthaltenden Gemisch, dadurch gekennzeichnet, daß die Rektifikation unter Zusatz eines primären Amins und/oder dessen Salzen erfolgt und es sich bei der hydrophoben organischen Flüssigkeit um ein Gemisch bestehend aus einer Mischung aus 70 bis 75 Gew.-% Diphenylether und 25 bis 30 Gew.-% Diphenyl sowie, bezogen auf diese Mischung, 0,1 bis 25 Gew.-% o-Dimethylphthalat handelt.

2. Verfahren nach Anspruch 1, dadurch gekennzeichnet, daß das die (Meth)acrylsäure enthaltende Gemisch 5 bis 25 Gew.-% (Meth)acrylsäure enthält.

3. Verfahren nach Anspruch 1 oder 2, dadurch gekennzeichnet, daß als primäres Amin ein Hydrazinderivat zugesetzt wird.

4. Verfahren nach Anspruch 1 bis 3, dadurch gekennzeichnet, daß als primäres Amin das Hydrazid einer organischen Carbonsäure zugesetzt wird.

5. Verfahren nach Anspruch 1 bis 4, dadurch gekennzeichnet, daß als primäres Amin Adipinsäuredihydrazid zugesetzt wird.

6. Verfahren nach Anspruch 1 bis 5, dadurch gekennzeichnet, daß die rektifikative Abtrennung bei einem Druck von 10 bis 100 mbar durchgeführt wird.

7. Verfahren nach Anspruch 1 bis 6, dadurch gekennzeichnet, daß es kontinuierlich durchgeführt wird und der Aminzusatz in die Rektifikationskolonne unmittelbar unterhalb der Entnahmestelle der (Meth)acrylsäure erfolgt.

8. Verfahren nach Anspruch 1 bis 7, dadurch gekennzeichnet, daß die rektifikative Abtrennung im Beisein von Phenothiazin als Polymerisationsinhibitor durchgeführt wird.

9. Verfahren nach Anspruch 1 bis 8, dadurch gekennzeichnet, daß die abzutrennende (Meth)acrylsäure durch katalytische Gasphasenoxidation von C₃-/C₄-Ausgangsverbindungen erzeugt worden ist.

10. Verfahren zur Herstellung von (Meth)acrylsäure durch katalytische Gasphasenoxidation einer C₃-/C₄-Ausgangsverbindung, bei dem man das Reaktionsgasgemisch der Gasphasenoxidation in einer Absorptionskolonne zu einer absteigenden hochsiedenden inerten hydrophoben organischen Flüssigkeit im Gegenstrom führt, dem in der Absorptionskolonne in natürlicher Weise erfolgenden Absorptionsprozeß einen Rektifikationsprozeß dadurch überlagert, daß man der Absorptionskolonne eine über ihre, aufgrund ihres Kontaktes mit der Umgebungstemperatur erfolgende, natürliche Energieabgabe hinausgehende Energiemenge entzieht und aus dem Flüssigkeitsablauf der Absorptionskolonne die (Meth)acrylsäure rektifikativ abtrennt, dadurch gekennzeichnet, daß die rektifikative Abtrennung unter Zusatz eines primären Amins und/oder dessen Salzen erfolgt und es sich bei der hydrophoben organischen Flüssigkeit um ein Gemisch bestehend aus einer Mischung aus 70 bis 75 Gew.-% Diphenylether und 25 bis 30 Gew.-% Diphenyl sowie, bezogen auf diese Mischung, 0,1 bis 25 Gew.-% o-Dimethylphthalat handelt.

11. Verfahren zur Herstellung von (Meth)acrylsäure durch katalytische Gasphasenoxidation einer C₃-/C₄-Ausgangsverbindung, bei dem man das Reaktionsgasgemisch der Gasphasenoxidation in einer Absorptionskolonne zu einer absteigenden hochsiedenden inerten hydrophoben organischen Flüssigkeit im Gegenstrom führt, danach den Flüssigkeitsablauf der Absorptionskolonne in einer Desorptionskolonne mit Inertgas strippt und aus dem Flüssigkeitsablauf der Desorptionskolonne die (Meth)acrylsäure rektifikativ abtrennt, dadurch gekennzeichnet, daß die rektifikative Abtrennung unter Zusatz eines primären Amins und/oder dessen Salzen erfolgt und es sich bei der hydrophoben organischen Flüssigkeit um ein Gemisch bestehend aus einer Mischung aus 70 bis 75 Gew.-% Diphenylether und 25 bis 30 Gew.-% Diphenyl sowie, bezogen auf diese Mischung, 0,1 bis 25 Gew.-% o-Dimethylphthalat handelt.

12. Verfahren nach Anspruch 10 oder 11, dadurch gekennzeichnet, daß es sich um ein Verfahren zur Herstellung von Acrylsäure handelt und als Ausgangsverbindung der Gasphasenoxidation Propen und/oder Acrolein eingesetzt wird.

## Claims

1. A process for the separation by rectification of (meth)-acrylic acid from a mixture containing (meth)acrylic acid and an inert hydrophobic organic liquid having a boiling point higher than that of (meth)acrylic acid as main constituents and also lower aldehydes as secondary constituents, wherein the rectification is carried out with the addition of a primary amine and/or salts thereof and the hydrophobic organic liquid is a mixture comprising a mixture of from 70 to 75% by weight of diphenyl ether and from 25 to 30% by weight of biphenyl and, based on this mixture, from 0.1 to 25% by weight of o-dimethyl phthalate.

2. A process as claimed in claim 1, wherein the mixture containing the (meth)acrylic acid contains from 5 to 25% by weight of (meth)acrylic acid.

3. A process as claimed in claim 1 or 2, wherein the primary amine added is a hydrazine derivative.

4. A process as claimed in any of claims 1 to 3, wherein the primary amine added is the hydrazide of an organic carboxylic acid.

5. A process as claimed in any of claims 1 to 4, wherein the primary amine added is adipic acid dihydrazide.

6. A process as claimed in any of claims 1 to 5, wherein the separation by rectification is carried out at a pressure of from 10 to 100 mbar.

7. A process as claimed in any of claims 1 to 6, which is carried out continuously and in which the amine is added to the rectification column directly below the outlet point for the (meth)acrylic acid.

8. A process as claimed in any of claims 1 to 7, wherein the separation by rectification is carried out in the presence of phenothiazine as polymerization inhibitor.

9. A process as claimed in any of claims 1 to 8, wherein the (meth)acrylic acid to be separated off has been produced by catalytic gas-phase oxidation of C₃/C₄ starting compounds.

10. A process for preparing (meth)acrylic acid by catalytic gas-phase oxidation of a C₃/C₄ starting compound, in which the reaction gas mixture from the gas-phase oxidation is passed through an absorption column in countercurrent to a descending high-boiling inert hydrophobic organic liquid, a rectification process is superimposed on the absorption process occurring naturally in the absorption column by withdrawing from the absorption column an amount of energy greater than its natural energy loss resulting from its contact with the surroundings, and separating the (meth)acrylic acid by rectification from the liquid discharge from the absorption column, wherein the separation by rectification is carried out with addition of a primary amine and/or salts thereof and the hydrophobic organic liquid is a mixture comprising a mixture of from 70 to 75% by weight of diphenyl ether and from 25 to 30% by weight of biphenyl and, based on this mixture, from 0.1 to 25% by weight of o-dimethyl phthalate.

11. A process for preparing (meth)acrylic acid by catalytic gas-phase oxidation of a C₃/C₄ starting compound, in which the reaction gas mixture from the gas-phase oxidation is passed through an absorption column in countercurrent to a descending high-boiling inert hydrophobic organic liquid, the liquid discharge from the absorption column is then stripped in a desorption column using inert gas, and the (meth)acrylic acid is separated by rectification from the liquid discharge of the desorption column, wherein the separation by rectification is carried out with addition of a primary amine and/or salts thereof and the hydrophobic organic liquid is a mixture comprising a mixture of from 70 to 75% by weight of diphenyl ether and from 25 to 30% by weight of biphenyl and, based on this mixture, from 0.1 to 25% by weight of o-dimethyl phthalate.

12. A process as claimed in claim 10 or 11, which is a process for preparing acrylic acid and the starting compound used for the gas-phase oxidation is propene and/or acrolein.

## Revendications

1. Procédé de séparation par rectification d'acide (méth)acrylique à partir d'un mélange contenant en tant que constituants principaux de l'acide (méth)acrylique et un liquide organique hydrophobe inerte dont le point d'ébullition est plus élevé que celui de l'acide (méth)acrylique, et, en tant que constituants secondaires, des aldéhydes inférieurs, caractérisé en ce que la rectification est effectuée par addition d'une amine primaire et/ou de ses sels, et que, pour ce qui concerne le liquide organique hydrophobe, il s'agit d'un mélange constitué d'un mélange de 70 à 75 % en poids de diphényléther et de 25 à 30 % en poids de diphényle, et aussi, par rapport à ce mélange, de 0,1 à 25 % en poids de phtalate d'o-diméthyle.

2. Procédé selon la revendication 1, caractérisé en ce que le mélange contenant de l'acide (méth)acrylique contient de 5 à 25 % en poids d'acide (méth)acrylique.

3. Procédé selon la revendication 1 ou 2, caractérisé en ce qu'on ajoute en tant qu'amine primaire un dérivé de l'hydrazine.

4. Procédé selon les revendications 1 à 3, caractérisé en ce qu'on ajoute en tant qu'amine primaire l'hydrazide d'un acide carboxylique organique.

5. Procédé selon les revendications 1 à 4, caractérisé en ce qu'on ajoute en tant qu'amine primaire le dihydrazide adipique.

6. Procédé selon les revendications 1 à 5, caractérisé en ce que la séparation par rectification est mise en oeuvre sous une pression de 10 à 100 mbar.

7. Procédé selon les revendications 1 à 6, caractérisé en ce qu'il est mis en oeuvre en continu, et que l'addition de l'amine dans la colonne de rectification a lieu immédiatement en dessous du point de soutirage de l'acide (méth)acrylique.

8. Procédé selon les revendications 1 à 7, caractérisé en ce que la séparation par rectification est mise en oeuvre en présence de phénothiazine en tant qu'inhibiteur de polymérisation.

9. Procédé selon les revendications 1 à 8, caractérisé en ce que l'acide (méth)acrylique à séparer a été produit par oxydation catalytique en phase gazeuse de composés de départ en C₃/C₄.

10. Procédé de préparation d'acide (méth)acrylique par oxydation catalytique en phase gazeuse d'un composé de départ en C₃/C₄, dans lequel on envoie à contre-courant le mélange de gaz de réaction de l'oxydation en phase gazeuse, dans une colonne d'absorption, dans un liquide organique hydrophobe inerte ascendant à haut point d'ébullition ; on superpose au processus d'absorption qui a lieu d'une manière naturelle dans la colonne d'absorption un processus de rectification, de façon à soutirer de la colonne d'absorption une quantité d'énergie supérieure à la quantité d'énergie s'évacuant d'une manière naturelle en conséquence de son contact avec la température ambiante, et on sépare par rectification l'acide (méth)acrylique du liquide sortant de la colonne d'absorption, caractérisé en ce que la séparation par rectification s'effectue avec addition d'une amine primaire et/ou de ses sels, et que, pour ce qui concerne le liquide organique hydrophobe, il s'agit d'un mélange constitué d'un mélange de 70 à 75 % en poids de diphényléther et de 25 à 30 % en poids de diphényle, et aussi, par rapport à ce mélange, de 0,1 à 25 % en poids de phtalate d'o-diméthyle.

11. Procédé de préparation d'acide (méth)acrylique par oxydation catalytique en phase gazeuse d'un composé de départ en C₃/C₄, dans lequel on envoie à contre-courant le mélange de gaz de réaction de l'oxydation en phase gazeuse, dans une colonne d'absorption, à un liquide organique hydrophobe inerte ascendant à haut point d'ébullition ; puis, avec un gaz inerte, on épuise dans une colonne de désorption le liquide sortant de la colonne d'absorption, et on sépare par rectification l'acide (méth)acrylique du liquide sortant de la colonne de désorption, caractérisé en ce que la séparation par rectification s'effectue avec addition d'une amine primaire et/ou de ses sels, et que, pour ce qui concerne le liquide organiqine hydrophobe, il s'agit d'un mélange constitué d'un mélange de 70 à 75 % en poids de diphényléther et de 25 à 30 % en poids de diphényle, et aussi, par rapport à ce mélange, de 0,1 à 25 % en poids de phtalate d'o-diméthyle.

12. Procédé selon la revendication 10 ou 11, caractérisé en ce qu'il s'agit d'un procédé de préparation d'acide acrylique, et que l'on utilise en tant que composé de départ de l'oxydation en phase gazeuse du propène et/ou de l'acroléine.
